# EUROPEAN PATENT APPLICATION

(11) **EP 1 918 870 A1**
(43) Date of publication of application: **07.05.2008**
(21) Application number: 06796602.8
(22) Date of filing: 21.08.2006
(51) Int. Cl.: G06Q 50/00, A61B 1/00, A61B 5/00

(54) **IMAGE DISPLAY APPARATUS**

(30) Priority: 22.08.2005 JP 2005240252; 09.09.2005 JP 2005263090
(71) Applicant: Olympus Corporation, Tokyo 151-0072 (JP); OLYMPUS MEDICAL SYSTEMS CORP., Shibuya-ku Tokyo 151-0072 (JP)
(72) Inventor: HIRAKAWA, Katsumi c/o Olympus Med. Systems Corp., Tokyo 151-0072 (JP); KIMOTO, Seiichiro c/o Olympus Med. Systems Corp., Tokyo 151-0072 (JP)
(74) Representative: von Hellfeld, Axel
(86) International application number: PCT/JP2006/316339
(87) International publication number: WO 2007/023771

(57) **Abstract**

An image display apparatus 4 includes a control unit 15 having an image display controller 15a and an image processing controller 15b so that an imaging period where an image of interest is present can be recognized easily. The image display controller 15a displays a time bar 27 as a time scale indicating an imaging period of a series of intra-subject images; and a landmark button 29 as an appending unit that appends marker information indicating an image of interest to a main display image 23 displayed in a main display area 22. Further, the image display controller 15a controls, based on the imaging time point of the image of interest appended with the marker information, to display a display area before the imaging point or a display area after the imaging point on the time bar 27 so as to be discriminable from other display areas on the time bar 27. The image processing controller 15b obtains image data stored in a portable recording medium 5 or a storage unit 14, outputs the image data to an image processor 13, and controls various image processing for the output image.

## Description

### TECHNICAL FIELD

The present invention relates to an image display apparatus for displaying a series of images captured at multiple time points, specifically to an image display apparatus which is suitable for displaying a series of intra-subject images captured by using a capsule endoscope inserted in the subject.

### BACKGROUND ART

In recent years, a swallowable capsule endoscope has been developed in the field of an endoscope. The capsule endoscope having an imaging function and a radio communication function is inserted from a mouth into a body of a subject for an observation, and travels to capture images of the inside of organs such as the stomach, the small intestine, and the large intestine according to their peristalsis until it is naturally excreted.

While the capsule endoscope travels inside the organs, image data captured by the capsule endoscope in the subject body is sequentially transmitted to the outside of the subject by a radio signal to be stored in a memory provided in a receiver placed outside of the subject, or displayed on a display provided to the receiver. A doctor, a nurse, or the like can make a diagnosis based on images displayed on a display based on the image data stored in the memory, or images displayed on the display provided to the receiver simultaneously with the receiver's data reception.

Since the number of a series of images captured by the capsule endoscope is usually enormous, the doctor, nurse, or the like needs a great amount of time and effort to observe the enormous number of images and make a diagnosis. In response to such a circumstance, an image display apparatus, which has an improved image search performance and enables an easy recognition of what organ the image on the display captures, has been proposed (see Patent Document 1, for example).

The image display apparatus displays a time scale which indicates an imaging period of the series of images, and also displays time-series average colors of respective images on the time sale. Since the average colors of the images correspond to captured-organ-specific colors, respectively, the doctor, nurse, or the like can easily recognize, by observing the average color displayed on the time scale, what organ the images at respective imaging time points capture.

Patent Document 1: Japanese Patent Application Laid-Open No. 2004-337596

### DISCLOSURE OF INVENTION

### PROBLEM TO BE SOLVED BY THE INVENTION

However, the conventional image display apparatus described above, though it is possible to recognize the captured organ according to the imaging time point by displaying the time-series average colors of respective images on the time scale, cannot display images of interest which an observer or the like desires to see discriminatively on the time scale.

In view of the foregoing, an object of the present invention is to provide an image display apparatus which enables a discriminative display of an arbitrary image of interest on the time scale, and an easy recognition of an imaging period when the image of interest is present.

### MEANS FOR SOLVING PROBLEM

To solve the problems described above and achieve the object, an image display apparatus according to the invention as set forth in claim 1 for displaying a series of images captured at multiple time points and a time scale indicating an imaging period of the series of images, includes an appending unit that appends marker information indicating an image of interest to a predetermined image among the series of images; a display controller that controls to display one of display areas on the time scale corresponding to before and after an imaging time point of the image of interest to which the marker information is appended, and to display other display areas on the time scale so as to be discriminable from the one of the display areas.

In the image display apparatus according to the invention as set forth in claim 2, the display controller controls to display a display area on the time scale corresponding to between imaging time points of images of interest to each of which the marker information is appended, and to display other display areas on the time scale so as to be discriminable from the display area, when a plurality of images of interest to each of which the marker information is appended are present.

In the image display apparatus according to the invention as set forth in claim 3, the display controller controls to display the display areas on the time scale, each of the display areas, corresponding to before and after the imaging time point of the image of interest, having different at least one of hue, color saturation, luminance, pattern, shape, and size thereof.

In the image display apparatus according to the invention as set forth in claim 4, the display controller displays respective images of interest appended with the marker information as thumbnails, and controls to display relative times corresponding to imaging time points of thumbnails in the neighborhood of the displayed thumbnails.

In the image display apparatus according to the invention as set forth in claim 5, the display controller controls to display the relative time based on an imaging time point of a reference thumbnail as a reference, the reference thumbnail being selected among the thumbnails.

The image display apparatus according to the invention as set forth in claim 6 further includes a selection-information acquiring unit that acquires selection-information for selecting the reference thumbnail among the thumbnails, wherein the display controller controls to update the reference for each acquisition of the selection-information by the selection-information acquiring unit, and to display the relative time.

In the image display apparatus according to the invention as set forth in claim 7, the series of images are intra-subject images captured by using a capsule endoscope inserted into a subject.

### EFFECT OF THE INVENTION

The image display apparatus according to the present invention enables a discriminative display of an arbitrary image of interest on the time scale, and an easy recognition of an imaging period when the image of interest is present.

### BRIEF DESCRIPTION OF DRAWINGS

FIG. 1 is a schematic diagram of a configuration of a radio system for acquiring intra-subject information according to a first embodiment;
FIG. 2 is a block diagram of a configuration of an image display apparatus according to the first embodiment;
FIG. 3 is a diagram illustrating a display screen image of the image display apparatus shown in FIG. 1;
FIG. 4 is a diagram illustrating another display screen image of the image display apparatus shown in FIG. 1;
FIG. 5 is a flowchart of a procedure of a landmark setting operation to be performed by the image display apparatus shown in FIG. 1;
FIG. 6 is a diagram illustrating still another display screen image of the image display apparatus shown in FIG. 1;
FIG. 7 is a diagram illustrating still another display screen image of the image display apparatus shown in FIG. 1;
FIG. 8 is a diagram illustrating still another display screen image of the image display apparatus shown in FIG. 1;
FIG. 9 a schematic diagram of an entire configuration of a radio system for acquiring intra-subject information according to a second embodiment;
FIG. 10 is a schematic block diagram of a configuration of a display apparatus according to a second embodiment;
FIG. 11 is a flowchart for explaining a search operation of patient information performed by the display apparatus according to the second embodiment;
FIG. 12 is a flowchart for explaining a search operation of patient information performed by a display apparatus according to a modification of the second embodiment;
FIG. 13 is a schematic block diagram of a configuration of a filing system according to a third embodiment;
FIG. 14 is a flowchart for explaining a search operation of patient information performed by the filing system according to the third embodiment;
FIG. 15 is a schematic block diagram of a configuration of a filing system according to a fourth embodiment;
FIG. 16 is a flowchart for explaining a search operation of patient information performed by the filing system according to the fourth embodiment; and
FIG. 17 is a diagram illustrating one example of patient information displayed on a display unit of the display apparatus.

### EXPLANATIONS OF LETTERS OR NUMERALS

- 1: SUBJECT
- 2: CAPSULE ENDOSCOPE
- 3: RECEIVING DEVICE
- 4: IMAGE DISPLAY APPARATUS
- 5: PORTABLE RECORDING MEDIUM
- 6: RECEIVING ANTENNA
- 6a to 6h: ANTENNAS
- 11: INPUT UNIT
- 12: DISPLAY UNIT
- 13: IMAGE PROCESSOR
- 14: STORAGE UNIT
- 15: CONTROL UNIT
- 15a: IMAGE DISPLAY CONTROLLER
- 15b: IMAGE PROCESSING CONTROLLER
- 21: WINDOW
- 22: MAIN DISPLAY AREA
- 23: MAIN DISPLAY IMAGE
- 24: ANTENNA ARRANGEMENT PLAN
- 25: IMAGE OPERATION AREA
- 26: COLOR BAR
- 26a to 26d: DIVIDED COLOR BARS
- 27: TIME BAR
- 27a: SLIDER
- 27b to 27d: MARKERS
- 28: DISPLAY SUB-AREA
- 28a,: 28a-n THUMBNAILS
- 28b,: 28b' TEXTUAL INFORMATION
- 28c: SCROLL BAR
- 29: LANDMARK BUTTON
- 30: LANDMARK SETTING DIALOG BOX
- 101: SUBJECT
- 102: RECEIVING DEVICE
- 102a: RECEIVING JACKET
- 102b: EXTERNAL DEVICE
- 103: CAPSULE ENDOSCOPE
- 104: DISPLAY DEVICE
- 105: COMMUNICATION CABLE
- 106: SERVER
- 120, 121: INPUT UNITS
- 130, 131: DATABASES
- 140, 141: DISPLAY UNITS
- 142: DISPLAY AREA
- 143: CONFIRMATION BUTTON
- 150, 170: CONTROL UNITS
- 151, 171: SEARCH UNITS
- 152: SELECTOR
- 153: CONFIRMATION CONTROLLER
- 160, 161: INTERFACES
- A1 to An: ANTENNAS

### BEST MODE(S) FOR CARRYING OUT THE INVENTION

Exemplary embodiments of a radio system for acquiring intra-subject information as a preferred embodiment of an image display apparatus according to the present invention will be explained in detail with reference to the accompanying drawings. However, the present invention shall not be limited to the embodiments. Throughout the drawings, the same part is denoted by the same numeral.

### (First embodiment)

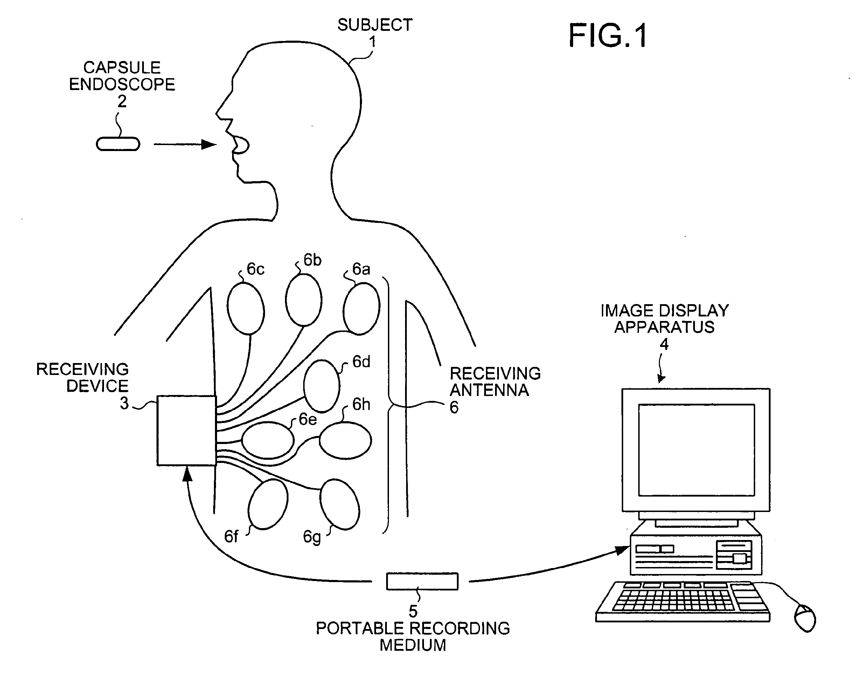

First, a radio system for acquiring intra-subject information provided with an image display apparatus according to a first embodiment will be explained. FIG. 1 is a schematic diagram of an entire configuration of the radio system for acquiring intra-subject information. The radio system for acquiring intra-subject information uses a capsule endoscope as one example of a body-insertable device.

As shown in FIG. 1, the radio system for acquiring intra-subject information includes a capsule endoscope 2 which is inserted into a body of a subject 1 to wirelessly transmit image data of a captured intra-subject image to a receiving device 3; the receiving device 3 which receives the image data wirelessly transmitted from the capsule endoscope 2; an image display apparatus 4 which displays the intra-subject image based on an image signal received by the receiving device 3; and a portable recording medium 5 which transfers image data and the like between the receiving device 3 and the image display apparatus 4.

The receiving device 3 include a receiving antenna 6 having a plurality of antennas 6a to 6h which are attached on an outside surface of the subject 1. The receiving device 3 receives image data and the like wirelessly transmitted from the capsule endoscope 2 via the receiving antenna 6, and records every piece of the received image data so as to associate with reception intensity information of respective antennas 6a to 6h at the time of data reception.

The antennas 6a to 6h realized by a loop antenna for example, are disposed at predetermined positions on the outside surface of the subject 1, i.e., positions respectively corresponding to organs as a path of the capsule endoscope 2 inside the subject 1. The antennas 6a to 6h may be arranged at predetermined positions on a jacket or the like to be worn by the subject 1. In this case, the antennas 6a to 6h are arranged at predetermined positions on the outside surface of the subject 1 through the jacket or the like. An arrangement of the antennas 6a to 6h may be changed arbitrarily depending on the purposes such as an observation, a diagnosis, or the like of the subject 1. The number of antennas provided to the receiving antenna 6 is not necessarily limited to eight as explained here as antennas 6a to 6h, and may be less or more than eight.

The image display apparatus 4 realized by a work station having a cathode-ray tube (CRT), a liquid crystal display, or the like for example, displays an image based on image data obtained via the portable recording medium 5 or the like. The image display apparatus 4 may output the image data to an output device such as a printer. The image display apparatus 4 has a function of communicating with an external device, and obtains/outputs the image data via wired or radio communication.

The portable recording medium 5 realized by a compact flash (registered trademark) memory, CD, DVD and the like, is detachable with respect to the receiving device 3 and the image display apparatus 4, and can record or output various types of information such as the image data and the like when the portable recording medium 5 is attached to the receiving device 3 and the image display apparatus 4. For example, the portable recording medium 5 is attached to the receiving device 3 and records the image data and the like transmitted from the capsule endoscope 2 to the receiving device 3, while the capsule endoscope 2 travels inside the subject 1. After the capsule endoscope 2 is discharged from the subject 1, the portable recording medium 5 is removed from the receiving device 3 and attached to the image display apparatus 4 to output the recorded image data and the like to the image display apparatus 4. Since the image data is transferred between the receiving device 3 and the image display device 4 via the portable recording medium 5, the subject 1 can freely move while the capsule endoscope 2 is in the subject 1. The image data may be transferred through wired or radio communication between the receiving device 3 and the image display apparatus 4.

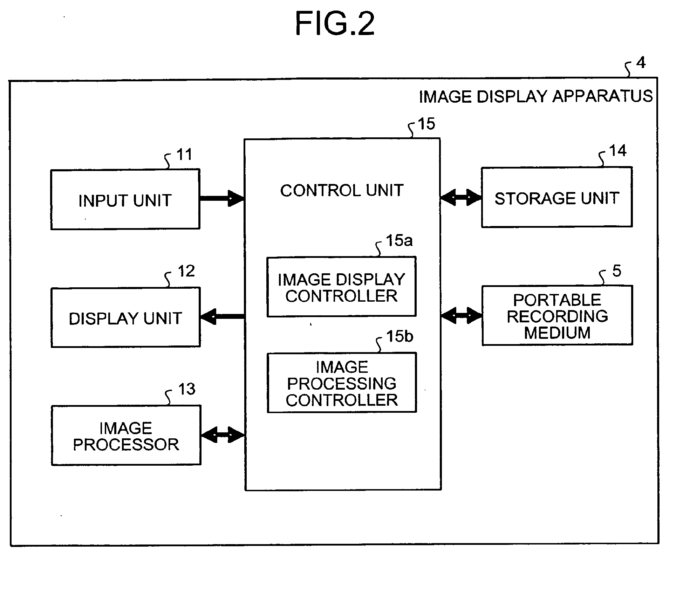

Next, a configuration of the image display apparatus 4 according to the first embodiment will be explained. FIG. 2 is a block diagram of a configuration of the image display apparatus 4. As shown in FIG. 2, the image display apparatus 4 includes an input unit 11 which allows inputting various types of information; a display unit 12 which displays the various types of information; an image processor 13 which processes the input image; a storage unit 14 which stores the various types of information; and a control unit 15 which controls the processing and operation of each unit of the image display apparatus 4. The input unit 11, the display unit 12, the image processor 13, and the storage unit 14 each are electrically connected to the control unit 15. The image display apparatus 4 further includes an interface for the portable recording medium 5 so that the portable recording medium can be detachably equipped. The portable recording medium 5 is electrically connected to the control unit 15 when attached to the image display apparatus 4.

The input unit 11 includes various switches, an input key, a mouse, a touch screen, and the like, and inputs various types of information such as selection-information of an image to be displayed. An observer of the displayed image as an operator of the image display apparatus 4 performs various operations of reading the displayed image, image selection, image recording and the like via the input unit 11. The input unit 11 may include an interface for wired or wireless communication such as a universal serial bus (USB), IEEE1394, or the like so that images can be input by an external device.

The display unit 12 includes a liquid crystal display and the like, and displays various types of information such as image data. Particularly, the display unit 12 displays various data such as image data stored in the portable recording medium 5 or the storage unit 14, and the Graphical User Interface (GUI) window which requests the observer or the like of the image display apparatus 4 to input various types of processing information.

The storage unit 14 is realized by a ROM in which various processing programs and the like are stored in advance, and a RAM which stores processing parameters for each processing, processing data, and the like. The storage unit 14 can store image data input via the portable recording medium 5 and the like, image data processed by the image processor 13, display control data processed by an image display controller 15a, and the like.

The image processor 13 obtains image data from the portable recording medium 5 or the storage unit 14 based on a control by an image processing controller 15b, and performs various image processing on the obtained image data, such as a concentration conversion (gamma conversion and the like), a smoothing (noise elimination and the like), a sharpening (edge emphasis and the like), an image recognition (detection of featured-image area, computing of an average color, and the like), and the like.

The control unit 15 is realized by a central processing unit (CPU) and the like which execute various processing programs stored in the storage unit 14. Specifically, the control unit 15 includes the image display controller 15a and the image processing controller 15b. The image display controller 15a controls to display a series of images captured at multiple time points as image data stored in the portable recording medium 5 or the storage unit 14 on the display unit 12. As the series of images specifically in the first embodiment, a series of images which capture the inside of organs of the subject 1 at multiple time points are displayed.

Specifically, the image display controller 15a controls to display a time scale indicating an imaging period of the series of the intra-subject images, and to display an operation icon as an appending unit that appends marker information indicating that a main display image displayed in a predetermined main display area among the series of the intra-subject images is an image of interest. The operation icon is, for example, displayed as an operation button on the GUI screen.

Based on the imaging time point of the image of interest to which the marker information is appended, the image display controller 15a further controls to display on the time scale a display area which represents a time before an imaging time point of the image of interest or a time after the imaging time point of the image of interest, so as to be discriminable from other display areas on the time scale. Here, a determination of which to display either the display area before the imaging time point or the display area after the imaging time point depends on the type of the marker information appended to the image of interest. When there are multiple images of interest to which the marker information is appended, the image display controller 15a controls to display a display area between imaging time points of respective images of interest so as to be discriminable from other display areas on the time scale.

For realizing the discriminative display, the image display controller 15a controls to display at least one of hue, color saturation, luminance, design (pattern), shape, and size of a desired display area on the time scale so as to be discriminable from the other display areas. Here, the desired display area to be discriminated from the other display areas on the time scale is one of two areas which are divided by an imaging time point of an image of interest on the time scale. Specifically, the image display controller 15a controls to discriminably display the display area before the imaging time point of the image of interest and the display area after the imaging time point of the image of interest by differentiating at least one of hue, color saturation, luminance, design (pattern), shape, and size on the time scale.

The image display controller 15a controls to display an image of interest to which the marker information is appended, as a thumbnail in a display sub-area separately from the main display area, and further controls to display a relative time corresponding to an imaging time point of each thumbnail near the displayed thumbnails each. In this case, the image display controller 15a can control to display relative times of respective thumbnails based on an imaging time point of a reference thumbnail which is arbitrarily selected from thumbnails displayed in the display sub-area.

The image display controller 15a controls to display an operation icon and the like as a selection-information acquiring unit that acquires selection-information for selecting the reference thumbnail. The operation icon is, for example, displayed as an operation button for exclusive use on the GUI screen, or an invisible operation button overlapped with the thumbnail. A clicking operation on the operation icon by using a mouse provided to the input unit 11, for example, executes inputting predetermined selection-information. The information display controller 15a can also control to update the reference whenever selection-information is acquired according to the execution of clicking on the operation icon and the like, i.e., whenever selection-information is updated, and to display a relative time based on an updated reference imaging time point.

The image processing controller 15b obtains image data stored in the portable recording medium 5 or the storage unit 14 to output to the image processor 13, and controls various image processing of the output image. The image processing controller 15b outputs the image data which is the result of processing in the image processor 13 to the storage unit 14 or the portable recording medium 5 for storage.

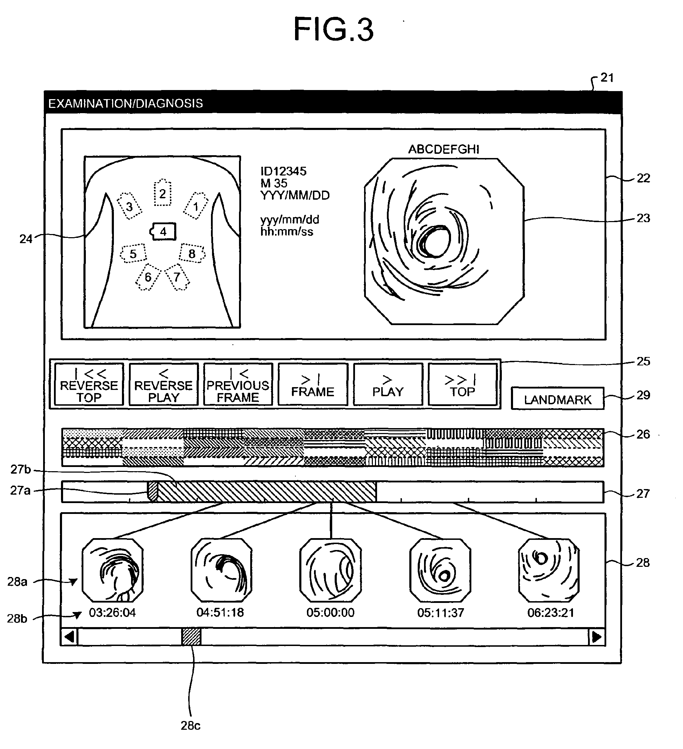

Next, the display screen (GUI screen) which is displayed on the display unit 12 in the image display apparatus 4 will be explained. FIG. 3 is a diagram illustrating one example of the GUI screen displayed based on a control by the image display controller 15a in the image display apparatus 4. As shown in FIG. 3, the display unit 12 displays a window 21 ("Examination/Diagnosis" window) as the GUI screen. The window 21 includes a main display area 22 which displays a main display image and the like; an image operation area 25 which has various image operation buttons shown as an icon; a color bar 26 and a time bar 27 as a time scale indicating an imaging period of the series of intra-subject images; and a display sub-area 28 which exhibits a thumbnail and the like, each being arranged from top to bottom in parallel according to the order described above.

The main display area 22 exhibits a main display image 23 which is an image selected from the series of intra-subject images based on instruction information input by the input unit 3; and an antenna arrangement plan 24 which schematically illustrates an arrangement of the antennas 6a to 6h on the outside surface of the subject 1. The main display area 22 further includes textual information of name, ID number, sex, age, birth date, imaging date, imaging time, and the like of the subject 1, which are associated with the intra-subject image selected as the main display image 23. The main display area 22 can house predetermined two or more number of main display images according to a predetermined operation.

The antenna arrangement plan 24 schematically illustrates an arrangement of the antennas 6a to 6h together with a partial contour of the subject 1. In the antenna arrangement plan 24, an antenna number as an identification of each antenna is shown near each of the antennas 6a to 6h. In FIG. 3, numerals "1" to "8" are denoted for the antenna number, for example. In the antenna arrangement plan 24, the antenna which has maximum reception intensity among the antennas 6a to 6h when the intra-subject image displayed as the main display image 23 is captured, is exhibited discriminably from the other antennas. FIG. 3 is a diagram illustrating a state where, as an antenna having maximum reception intensity, the antenna denoted by "4" is shown discriminably from the other antennas, for example. To realize a discriminative display, the image display controller 15a can control to display at least one of luminance, hue, and color saturation, of the antenna having the maximum reception intensity so as to be discriminable from the other antennas.

In the color bar 26, average colors of images in the series of intra-subject images are exhibited respectively in the time-series order as a whole. Specifically, a display area of each imaging time point on the color bar 26 indicates an average color of each intra-subject image captured at each imaging time point. Since the series of intra-subject images have organ-specific average colors respectively, the observer or the like can recognize the organ captured in the intra-subject image of each imaging time point based on the transition of the average colors along the time axis (lateral axis in FIG. 3) on the color bar 26.

The color bar 26 has a format where the entire display area thereof is divided into four in the lateral direction on the display screen. Divided color bars of respective divided levels indicate time-series average area-colors or time-series average period-area colors on respective levels, which respectively correspond to divided image areas of the series of intra-subject images. In other words, the average colors of respective intra-subject images are computed per each divided image area of the entire image area divided into four, and average area-colors or average period-area colors of respective divided image areas are displayed on the color bar 26 in an order corresponding to the divided order, for each of four divided scale areas which are separated as a result of division in the lateral direction of the display area of each time point.

According to the color bar 26, the observer or the like not only can recognize organs captured in intra-subject images at multiple time points, respectively based on the transition in the average colors along the time axis of the divided color bar of each divided level, but also can easily estimate the detailed condition inside the captured organ depending on the divided image areas. Accordingly, when an average color of a red color group is visually recognized on a divided color bar 26a which is the top level of four levels for a certain period, for example, the observer or the like can recognize that a bleeding portion is present inside the organ whose image is captured in the period, the bleeding portion is present within the imaged range corresponding to the divided image areas on the top level of all intra-subject images in the period, and the like. Moreover, when an average color of a black color group in an image area including the luminal portion is displayed on a level of the divided color bars different from the level on which an average color of the other image areas is displayed, the observer or the like can recognize the condition of the inside of organs within the imaged range excluding a luminal portion.

A slider 27a which is movable in the time axis direction is displayed on the time bar 27. The slider 27a indicates an imaging time point of an intra-subject image displayed as a main display image 23 on the time bar 27, and moves on the time bar 27 in response to a changeover of the main display image 23. For example, when any one of image operation buttons in the image operation area 25 is operated via a mouse and the like (not shown), an image displayed as the main display image 23 is changed from one to another, and then the slider 27a moves to a position indicating the imaging time point of the intra-subject image displayed as the main display image 23 after the changeover.

In contrast, when the slider 27a is operated to move by the mouse and the like, an intra-subject image corresponding to an imaging time point which is indicated by the moved slider 27a is displayed as the main display image 23. When the slider 27a is operated to move in a row, images are each changed and displayed as the main display image 23 in a row correspondingly to the operations. According to the slider 27a, the observer or the like can operate to move the slider 27a to an imaging time point corresponding to an intra-subject image of a desired organ which is picked out with reference to the color bar 26, so that the intra-subject image can be displayed immediately as the main display image 23.

Further, a maker 27b for indicating an imaging period of a group of images each recognized as an image of interest among the series of intra-subject images is displayed discriminably from the other display areas on the time bar 27. In FIG. 3, for example, the marker 27b is displayed in a color different from the color for the other display areas, so that the observer or the like can visually and easily recognize the imaging period of the group of images of interest.

A start time point (time point at the left end of the marker 27b) and an end time point (time point at the right end of the marker 27b) of the imaging period indicated by the marker 27a are set by an operation of a landmark button 29 serving as an operation icon for appending marker information to an intra-subject image. Specifically, an intra-subject image at an imaging time point which is set to the start time point of the marker 27b is displayed as the main display image 23. Then, the intra subject image as the main display image 23 is appended with marker information indicating the start time point by executing a clicking operation or the like on the landmark button 29 via the mouse (not shown). In the same manner, an intra-subject image at an imaging time point which is set to the end time point of the marker 27b is displayed as the main display image 23, and marker information indicating the end time point is appended to the image displayed as the main display image 23. When the start time point and end time point are set in such a manner, the marker 27b is displayed to clearly indicate the designated imaging period.

According to the marker 27b, the observer or the like can easily recognize that the intra-subject images within the imaging period designated by the marker 27b are the images of interest which should specially be paid attention to. Since information of the marker 27b, i.e., marker information indicating the start and end time points of the marker 27b is recorded so as to be associated with intra-subject images, the imaging period in which an image of interest is present can be displayed whenever the series of intra-subject images are displayed. Accordingly, it is possible to reduce the time and effort the observer or the like requires for image search, and to perform an observation of images of interest effectively.

The left ends of the color bar 26 and the time bar 27 as serving as a time scale, indicate an imaging time point of the first image of the time-series intra-subj ect images. The right ends thereof indicate an imaging time point of the last image of the time-series intra-subject images. Normally, the imaging time point at the left end corresponds to a start time point of image data reception by the receiving device 3, and the imaging time point at the right end corresponds to an end time point of the image data reception.

In the display sub-area 28, an image selected and extracted from the series of intra-subject images is displayed as a thumbnail 28a. Specifically, the intra-subject image displayed as the main display image 23 according to a predetermined button operation or mouse operation is additionally displayed in the display sub-area 28 as the thumbnail 28a.

In the display sub-area 28, each thumbnail has individual additional information displayed in the neighborhood as textual information 28b. As the textual information 28b, an imaging time of each of the displayed thumbnails, a relative time which corresponds to each of the imaging time points based on a predetermined reference time point, and comments appended by the observer or the like are shown. In FIG. 3, for example, relative times corresponding to respective imaging time points of thumbnails based on the reference imaging time point of the first image of the time-series images are shown as the textual information 28b.

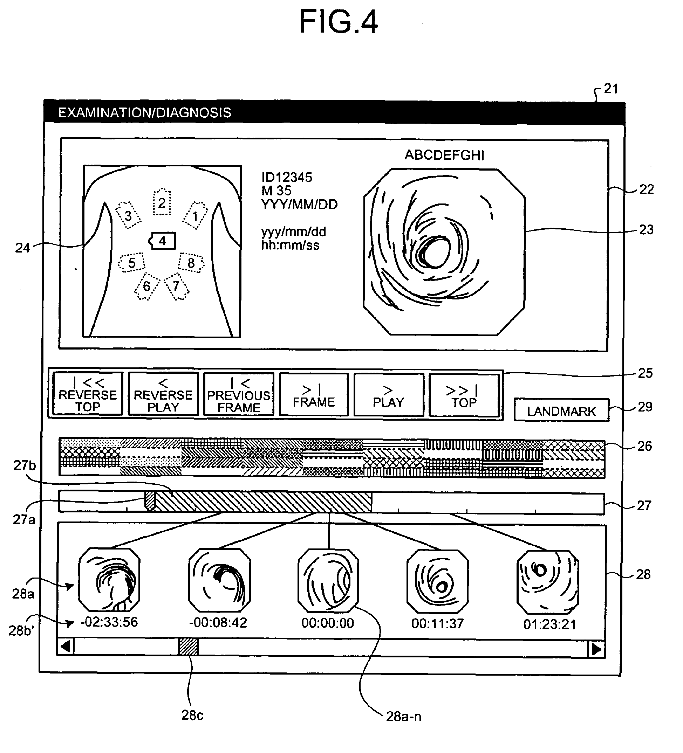

In such a relative time display, it is possible to change a reference time in accordance with a predetermined operation. Specifically for example, by clicking on any one of the displayed thumbnails, the imaging time point of the clicked thumbnail can be set as a reference for relative time. In a textual information 28b' in FIG. 4, for example, the reference for relative time (time "00:00:00") is changed to an imaging time point of thumbnail 28a-n as a result of clicking on the thumbnail 28a-n.

With a relative time display for each thumbnail, the observer and the like can estimate an intra-subject-imaging position of the thumbnail of interest. Specifically, when images capturing the diseased region and the like are observed based on a reference imaging time point of an intra-subject image capturing the small intestine, for example, the position of the diseased region can be calculated based on the start position of capturing the small intestine, and the relative time of the image capturing the diseased region.

The information content to be displayed as the textual information 28b or 28b' in the display sub-area 28 is variable according to a predetermined operation. It is also possible to hide the textual information 28b or 28b'. The display sub-area 28 includes lines which associate thumbnails and imaging time points of the thumbnails shown on the time bar 27, respectively.

Since there is a limitation in the display area available for the display sub-area 28, a batch display with up to a predetermined number of thumbnails 28a can be allowed. FIG. 3, for example, illustrates a case where a batch display with up to five thumbnails is allowed. When the number of extracted thumbnails 28a is greater than the predetermined number for the batch display, thumbnails over the predetermined number replace currently displayed thumbnails and are displayed in response to the operation of the scroll bar 28c displayed in the display sub-area 28. Each thumbnail displayed in the display sub-area 28 is displayed as the main display image 23 in response to the predetermined button operation or mouse operation.

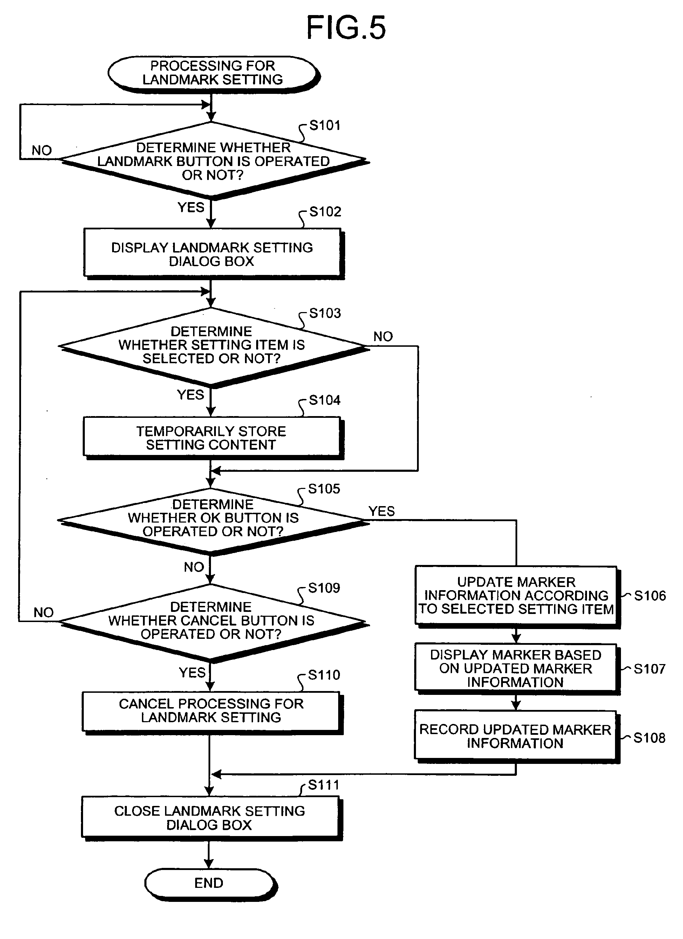

Here, a procedure for setting a landmark will be explained. The procedure is for displaying the marker on the time bar 27 in the image display apparatus 4 according to the first embodiment. FIG. 5 is a flowchart of the procedure for setting a landmark. As shown in FIG. 5, the image processing controller 15b determines whether a landmark button 29 is operated or not (step S101) to start the processing for the landmark setting. When the landmark button 29 is not operated ("No" at step S101), the determination processing is repeated in a predetermined cycle.

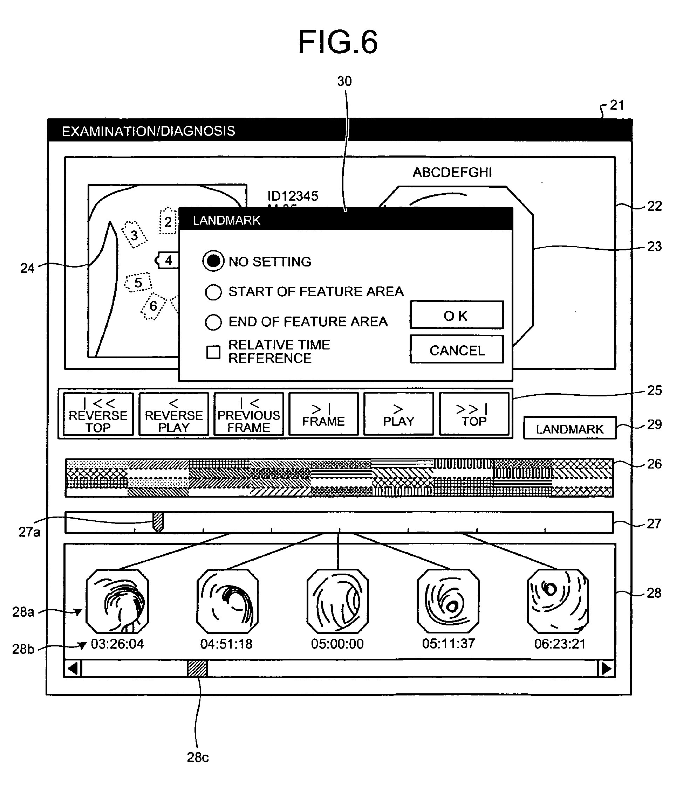

When the landmark button 29 is operated ("Yes" at step S101), the image display controller 15a displays a landmark setting dialog box for acquiring the detail of the marker information (step S102). At step S102, the image display controller 15a controls to display a landmark setting dialog box 30 so as to override the window 21, for example as shown in FIG. 6.

In the landmark setting dialog box 30 shown in FIG. 6, "START OF FEATURE AREA" as an item for setting marker information which designates a start time point of the marker shown on the time bar 27, "END OF FEATURE AREA" as an item for setting mark information which designates an end time point thereof, "NO SETTING" as an item for performing no setting, and "RELATIVE TIME REFERENCE" as an item for setting a reference for relative time which is appended to the thumbnail 28a as the textual information are present to allow a selection of any one of the items.

In the landmark setting dialog box 30, "OK" button for confirming the selected item, and "CANCEL" button for cancelling the setting operation with the landmark setting dialog box 30 are present. When the "OK" button or "CANCEL" button is operated, the landmark setting dialog box 30 is closed automatically after a predetermined processing.

Next, the image display controller 15a determines whether any setting item is selected on the landmark setting dialog box 30 or not (step S103). When any item is selected ("Yes" at step S103), settings of the selected item are temporarily stored (step S104). On the contrary, when any item is not selected ("No" at step S103), the process goes to step S105. The determination processing at step S103 may preferably be performed in a predetermined time passage after the execution of step S102.

The image display controller 15a then determines whether the "OK" button is operated on the landmark setting dialog box 30 or not (step S105). When the "OK" button is operated ("Yes" at step S105), marker information is updated depending on the selected setting item (step S106) and the marker based on the updated marker information is displayed on the time bar 27 (step S107). Then, the updated marker information is recorded in the storage unit 14 (step S108) to move to step S111.

On the contrary, when the "OK" button is not operated ("No" at step S105), the image display controller 15a determines whether the "CANCEL" button is operated on the landmark setting dialog box 30 or not (step S109). When the "CANCEL" button is not operated ("No" at step S109), the processing from step S103 is repeated. When the "CANCEL" button is operated ("Yes" at step S109), every processing for the landmark setting is cancelled (step S110), the landmark setting dialog box 30 is closed (step S111), and the series of landmark setting processing ends.

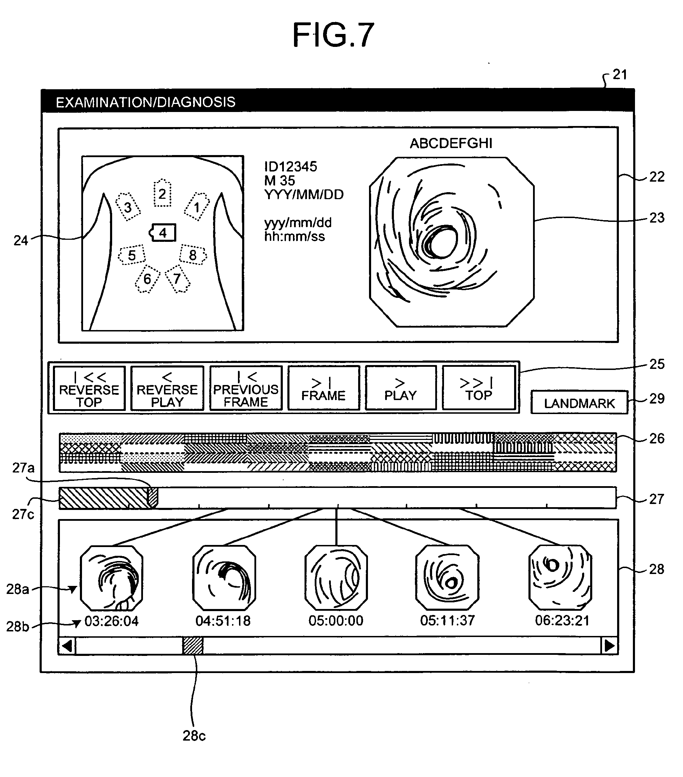

At step S107, when the selected item on the landmark setting dialog box 30 is "START OF FEATURE AREA", and there is no other intra-subject images associated with marker information in the series of intra-subject images, the image display controller 15a, for example as shown in FIG. 7, marks the display area before the imaging time point of the intra-subject image which is newly associated with marker information on the time bar 27 with a marker 27c.

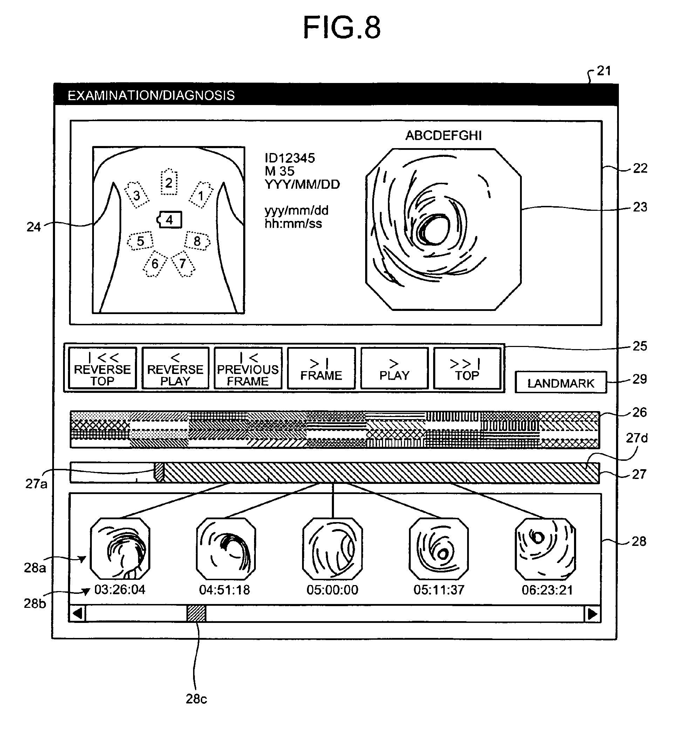

At step S107, when the selected item is "END OF FEATURE AREA", and there is no other intra-subject images associated with marker information in the series of intra-subject images, the image display controller 15a, for example as shown in FIG. 8, marks the display area after the imaging time point of the intra-subject image which is newly associated with marker information on the time bar 27 with a marker 27d.

At step S107, when the selected item is "START OF FEATURE AREA" (or "END OF FEATURE AREA"), and there is an intra-subject image associated with marker information which indicates an end time point (or a start time point) in the series of intra-subject images, the image display controller 15a, for example as shown in FIG. 3, marks the display area between respective pieces of marker information on the time bar 27 with the marker 27b.

As explained above, in the image display apparatus 4 according to the first embodiment, the markers 27b to 27d and the like each indicating an imaging period of a group of images which is recognized as an image of interest are present on the time bar 27 indicating the imaging period of the series of intra-subject images. Such markers are displayed so as to be discriminable from the other display areas on the time bar 27. Thus, the observer or the like can easily recognize the imaging period where images of interest are present, and thereby reducing the time and effort for searching images of interest in every observation and resulting in realizing effective observation of the images of interest.

In the first embodiment described above, the series of images displayed in the image display apparatus 4 according to the present invention are explained as the series of intra-subject images which are captured by using the capsule endoscope 2 inserted into the subject 1. However, it is not necessarily limited to the intra-subject images, and may be any images of any subject as long as a series of images are captured at multiple time points by using any imaging device.

### (Second embodiment)

Next, a radio system for acquiring intra-subject information according to a second embodiment will be explained. A display apparatus in this radio system for acquiring intra-subject information has a function as a filing device which stores patient information constituting of multiple kinds of information for specifying the subject (subject person, patient, and the like) in a storage unit as a database.

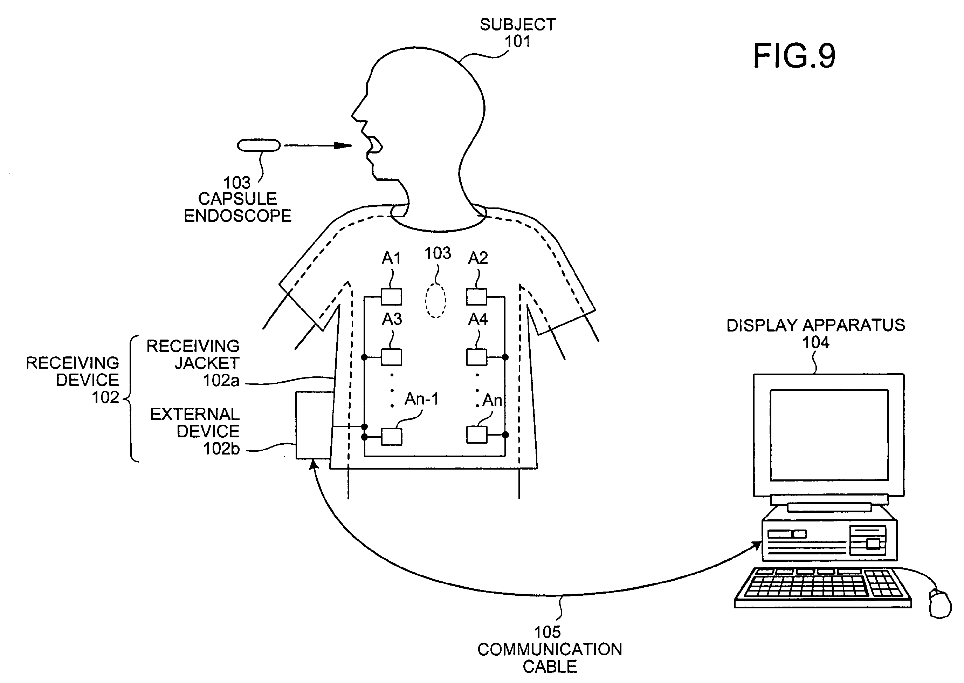

FIG. 9 is a schematic diagram of an entire configuration of the radio system for acquiring intra-subject information according to the second embodiment. The radio system for acquiring intra-subject information uses a capsule endoscope as one example of a body-insertable device. In FIG. 9, the radio system for acquiring intra-subject information includes a receiving device 102 which has a radio receiving function, a capsule endoscope (body-insertable device) 103 which is inserted into a subject 101, captures images inside the body cavity, and transmits data such as an image signal to the receiving device 102. The radio system for acquiring intra-subject information further includes a display apparatus 104 which displays a body cavity image based on the image signal received by the receiving device 102, and a communication cable 105 which transfers data between the receiving device 102 and the display apparatus 104. The receiving device 102 includes a receiving jacket 102a which is worn by the subject 101, and an external device 102b which performs processing and the like of radio signals received via a plurality of antennas A1 to An attached to the receiving jacket 102a.

The display apparatus 104 displays a body cavity image captured by the capsule endoscope 103, and has a configuration like a work station which displays an image based on data obtained from the receiving device 102 via the communication cable 105. Specifically, the display apparatus 104 may be configured to display directly on a CRT display, liquid crystal display, and the like, or may be configured to output an image to other media.

The communication cable 105 is normally detachable with respect to the external device 102b and the display apparatus 104. The external device 102b is configured to be capable of inputting/outputting or recording data information when the communication cable 105 is connected to both of the external device 102b and the display apparatus 104. In the second embodiment, when the receiving device 102 is initialized, for example, when old data such as image data stored in the storage unit in the receiving device 102 in a previous examination is deleted, or patient information is registered, the communication cable 105 is connected to the external device 102b and the display apparatus 104 to transmit data from the display apparatus 104 to the external device 102b. When the initialization is completed, the communication cable 105 is removed from the external device 102b and the display apparatus 104 to make the external device 102b and the display apparatus 104 unconnected. While the capsule endoscope 103 travels inside the body cavity of the subject 101, the external device 102b and the display apparatus 104 are kept unconnected with each other.

The external device 102b receives and records data wirelessly transmitted from the capsule endoscope 103. After the capsule endoscope 103 is discharged from the subject 101, i.e., after the imaging of the inside of the subject 101 is finished, the communication cable 105 is connected to the external device 102b and the display apparatus 104, so that the display apparatus 104 reads out the data which is transmitted by the capsule endoscope 103 and recorded by the external device 102b. The communication between the external device 102b and the display apparatus 104 according to the present invention is not limited to using the communication cable 105, and may be performed via wireless connection or may be performed by connecting the external device 102b and the display apparatus 104 with a cradle capable of data synchronization. In this case, the display apparatus and the cradle are connected through the communication cable, the external device 102b is disposed on the cradle, and then data transfer is performed between the external device 102b and the display apparatus 104. Patient information includes information such as an examination ID like an examination date, a name, an age, and a sex of the patient.

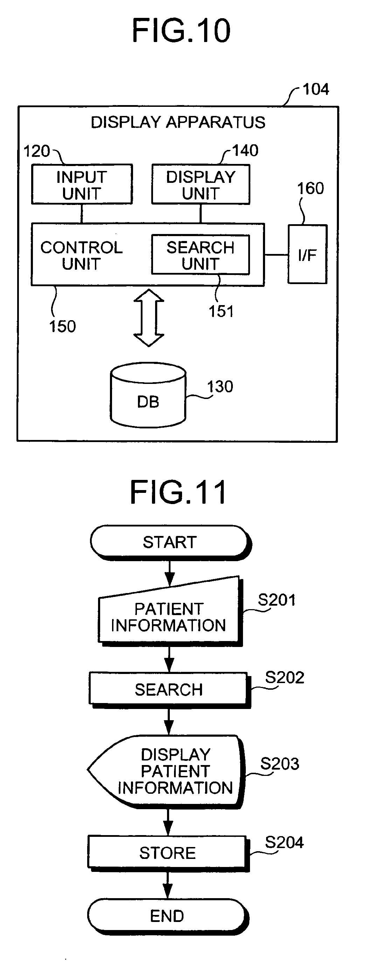

Next, the configuration of the display apparatus 104 will be explained. FIG. 10 is a schematic block diagram of the configuration of the display apparatus 104 according to the second embodiment. In FIG. 10, the display apparatus 104 includes an input unit 120 as an input unit; a database 130 as a storage unit; a display unit 140 as a display unit; a control unit 150; and an interface 160 as a connecting unit with other equipment, and has a function of filing data information such as patient information and image information.

The input unit 120 realized by a pointing device such as a keyboard and a mouse inputs information for instructing the operation of the display apparatus 104 and the processing to be performed by the display apparatus 104, and transmits the instruction information to the control unit 150. The input unit 120 also inputs selection-information for selecting a desired image from images displayed in a display area of the display unit 140. For example, when the mouse as the input unit 120 is operated to move a cursor displayed on the screen to the image displayed in the display area of the display unit 140, and the desired image is clicked on, the input unit 120 inputs instruction information as selection-information for selecting the desired image.

The input unit 120, for example by operating the keyboard, inputs patient information necessary for initialization of the receiving device 102, such as an examination ID, name, age, and sex of the patient, and the like, and transmits the patient information to the control unit 150. When the input unit 120 searches the patient information stored in the database 130, the input unit 120 inputs one piece of the patient information, for example, patient name to be transmitted to a search unit 151 of the control unit 150 described later.

The database 130 realized by a hard disc device and the like, for example, is capable of retaining various images and the like, storing patient information transmitted from the input unit 120, searching and reading of the information by the search unit 151.

The display unit 140 realized by the CRT display, liquid crystal display, and the like displays instruction information from the input unit 120 or instruction results. The display unit 140 displays patient information input by the input unit 120 and patient information searched by the search unit 151 based on one piece of patient information input by the input unit 120. The display unit 140 further displays a body cavity image of a group of images stored in the database 130, reduced-scale images (thumbnails) instructed by the instruction information, and the like.

The control unit 150 controls processing and operation of the input unit 120, database 130, and the display unit 140 each. The control unit 150 includes the search unit 151 which searches patient information stored in the database 130. The search unit 151 controls to search relevant patient information from patient information stored in the database and display on the display unit 140 based on the patient name of patient information input by an operation of the keyboard as the input unit 120 performed by a user such as a doctor. When there are plural patients having the same name, a plural pieces of patient information for plural patients are searched and displayed, so that the user can select, by operating the mouse of the input unit 120, the relevant patient information of the subject to be actually examined. The information to be a search key is not limited to name, and may be any one piece of information other than the patient name of the patient information, for example, an age. The interface 160 is an input/output interface for connecting the display apparatus 104 and another device, the receiving device 102, for example.

Next, an operation for searching patient information in the display apparatus 104 will be explained with reference to the flowchart in FIG. 11. FIG. 11 is a flowchart for explaining a search operation of patient information performed by the display apparatus 104 according to the second embodiment. In FIG. 11, when the user such as a doctor operates the keyboard of the input unit 120 and completes inputting one piece of the patient information, for example, patient name information (step S201), the search unit 151 searches the database 130 for patient information based on the input patient name (step S202).

Then, when the search unit 151 searches the relevant patient information, the patient information as the search result is displayed in the display area of the display unit 140 (step S203). When there is no relevant patient name input, all pieces of patient information including the patient name, age, sex, examination ID, and the like are input and stored in the database 130 (step S204).

The display apparatus 104 electrically connected to the external device 102b of the receiving device 102 through the communication cable 105, performs a data synchronization between the external device 102b and the display apparatus 104 to enable a data transfer. The external device 102b includes a hard disc (not shown) as an internal storage medium. Before the examination, the external device 102b and the display apparatus 104 are connected through the communication cable 105, and the searched patient information is transferred from the display apparatus 104 of the work station to the external device 102b to be stored in the hard disc.

While the capsule endoscope 103 travels inside the body cavity of the subject 101, the communication cable 105 is removed from the external device 102b, and then the external device 102b is attached to the subject 101 to record data transmitted from the capsule endoscope 103. After imaging of the inside of the subject 101 is finished, the external device 102b is again connected to the display apparatus 104 through the communication cable 105, and the display apparatus 104 reads out data (image information) recorded in the hard disc of the external device 102b.

In the second embodiment, since the search unit 151 searches the database 130 for the entirety of the relevant patient information at a stage where one piece of patient information is input and then controls to display the search result on the display unit 140, a labor of inputting the patient information can be saved with a quick search of the patient information stored in the database 130.

### (Modification)

In the second embodiment, patient information is searched at the stage where the user completes inputting one piece of the patient information. However, the search unit 151 may start searching in the middle of inputting one piece of the patient information, i.e., at a time when a part of one piece of the patient information is input. In the modification, patient information of a patient family name which is the same as the name previously searched right before the current search is controlled to be displayed on the display area of the display unit 140 by searching the database 130 at a time when the family name of the patient full name is input by the input unit 120. The patient information in the database 130 is appended with history information indicating the date of searching the patient information, for example.

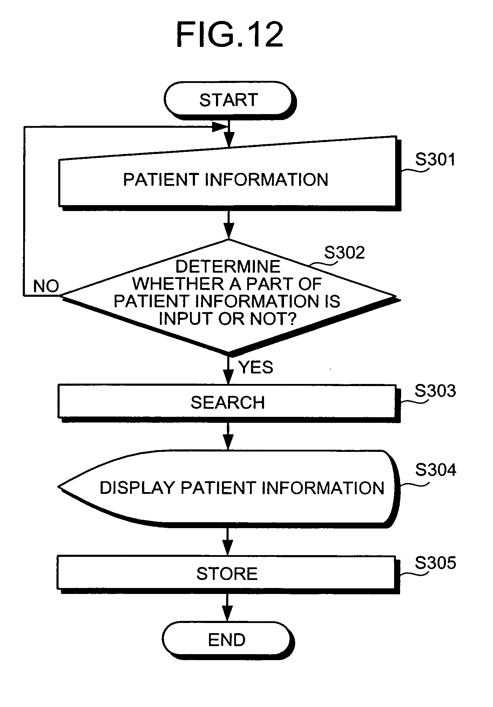

FIG. 12 is a flowchart of the modification of the second embodiment for explaining a search operation of patient information in the display apparatus 104. In this modification, a case where patient information of a patient named "Hanako Yamada" is, for example, searched will be explained (the same is applied to the other embodiments to be described below).

In FIG. 12, when the user inputs one piece of patient information "Hanako Yamada" by operating the keyboard of the input unit 120 (step S301), the search unit 151, at a time when "Yamada" is input (step S302), searches the database 130 for patient information which includes "Yamada" and is searched right before the current search based on history information (step S303), and displays the searched patient information in the display area of the display unit 140 (step 5304). In the modification, when there is no relevant patient name "Hanako Yamada", all pieces of patient information including the patient name, age, sex, examination ID, and the like are input and stored in the database 130 (step S305).

In this modification as described, since the search unit 151 searches the database 130 for relevant patient information at the time when a part of one piece of patient information is input, and then controls to display the search result on the display unit 140, a labor of inputting the patient information can be saved with a quicker search of the patient information stored in the database 130.

### (Third embodiment)

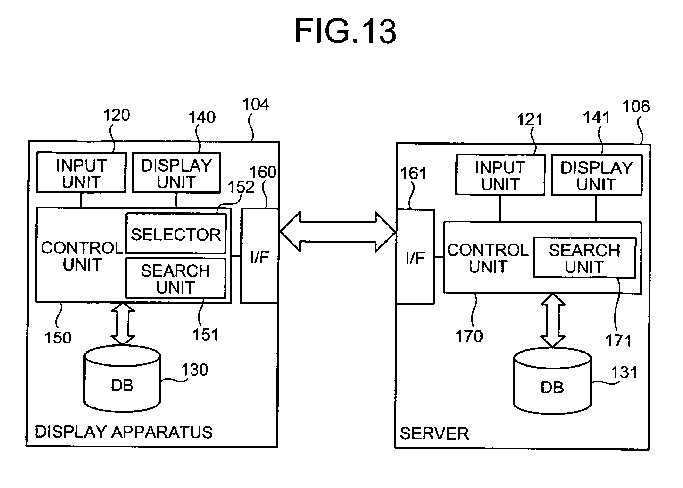

FIG. 13 is a schematic block diagram of a configuration of a filing system according to a third embodiment. In FIG. 13, a display apparatus 104 according to the third embodiment is different from the display apparatus according to the second embodiment in that a selector 152 is provided in the control unit 150 as a selector which selects a target database to be searched for patient information when there are a plurality of databases in a system, and that the display apparatus is connected to a server 106, which stores patient information, via the interface 160.

In the third embodiment, the display apparatus 104 constitutes a second filing device, and the server 106 constitutes a first filing device. The display apparatus 104 includes the input unit 120 as a second input unit having the same function as in the display apparatus according to the second embodiment; the database 130 as a second storage unit; the display unit 140 as a second display unit; the control unit 150; the search unit 151 as a second search unit; and the interface 160, other than the selector 152. The selector 152 selects a database which is searched for patient information with respect to the database 130 in the display apparatus and a database 131 in the server 106. In the third embodiment, the database 130 in the display apparatus having a higher hit rate in information search is selected first, and the database 131 is then selected when there is no relevant patient information found in the database 130.

The server 106 includes an input unit 121 as a first input unit having the same function as in the display apparatus 104 according to the second embodiment; the database 131 as a first storage unit; a display unit 141 as a first display unit; a control unit 170; a search unit 171 as a first search unit; and an interface 161. When the selector 152 selects the database 131 as a target for search, the search unit 171 searches the database 131 for relevant patient information based on a part of one piece of the patient information input by the input unit 120 to output the search result of patient information to the display apparatus 104 via the interface 161 (this is the same function as in the modification of the second embodiment) . Even when patient information is input by the input unit 121, the search unit 171 searches database 131 for the relevant patient information based on a part of one piece of the patient information, and controls to display the search result of patient information in the display area of the display unit 141.

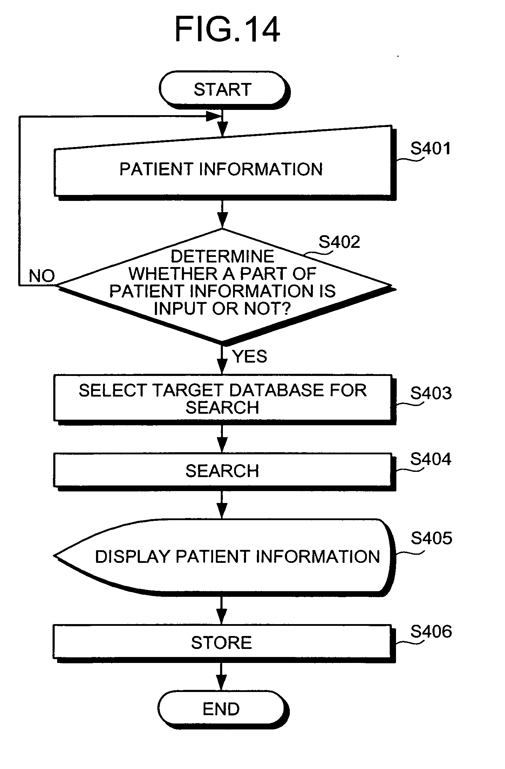

Next, an operation for searching patient information in a filing system will be explained with reference to the flowchart in FIG. 14. FIG. 14 is a flowchart for explaining a search operation of the patient information in the filing system according to the third embodiment. In FIG. 14, when the user inputs one piece of patient information "Hanako Yamada" by operating the keyboard of the input unit 120 (step S401), the search unit 151, at a time when input of "Yamada" is completed (step S402), the selector 152 selects the database 130 in the display apparatus as a target for search (step S403).

Next, in response to the selection of the database 130 by the selector 152, the search unit 151 searches the database 130 for patient information including the name of "Yamada", which is searched right before the current search, based on the history information (step S404), and displays the searched patient information in the display area of the display unit 140 (step S405).

Here, when the search unit 151 cannot find relevant patient information, the selector 152 selects the database 131 as a search target at step S403. This selection-information is transmitted to the server 106 via the interface 160. Then, in response to the selection of the database 131 by the selector 152, the search unit 171 of the server 106 searches the database 131 for patient information including the name of "Yamada", which is searched right before the current search, based on the history information (step S404), and transmits the search result of patient information to the display apparatus 104 via the interface 161.

When the search unit 151 of the display apparatus 104 retrieves the patient information, the searched patient information is displayed in the display area of the display unit 140 (step S405), and stores the patient information in the database 130 (step S406). In the third embodiment, when there is no relevant patient name "Hanako Yamada", the server 106 transmits the search result that there is no targeted information to the display apparatus 104, for example, and then the control unit 150, based on the search result, controls to input all pieces of patient information such as the patient name, age, sex, examination ID, and the like and to store the information in the database 130 (step S406).

In the third embodiment as described, when there are a plurality of databases in the system, patient information is searched after the selector selects a target database for search. Thus, it is possible to securely retrieve necessary patient information from the plurality of databases with the same advantages as in the second embodiment.

### (Fourth embodiment)

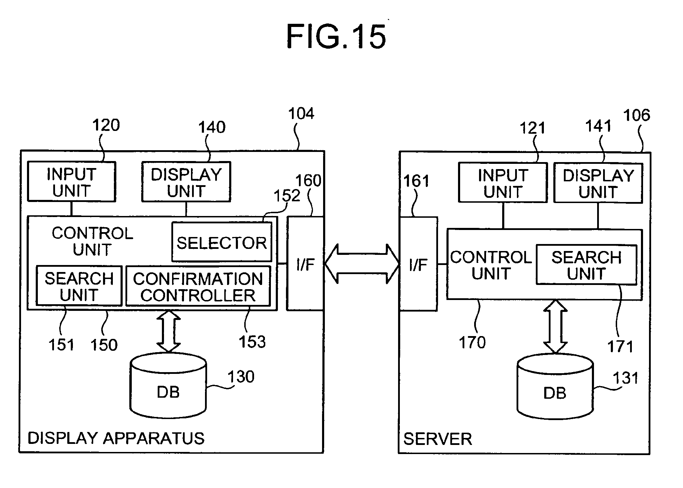

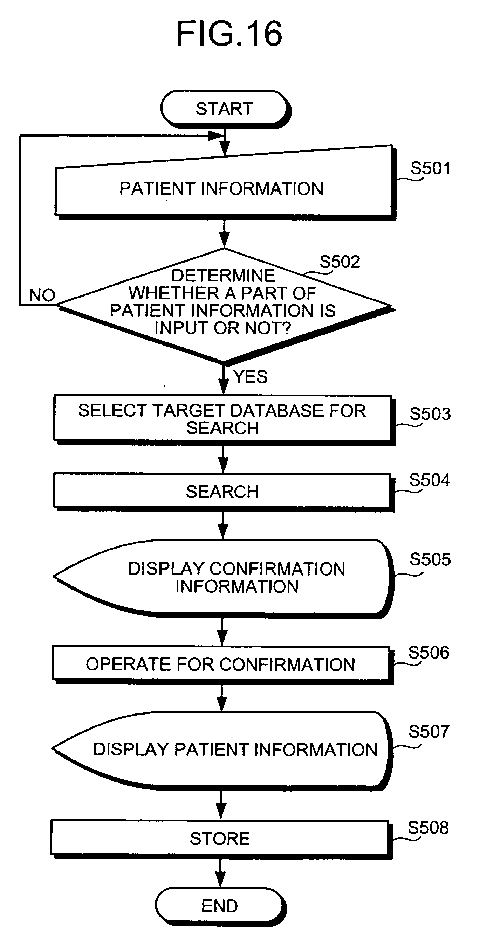

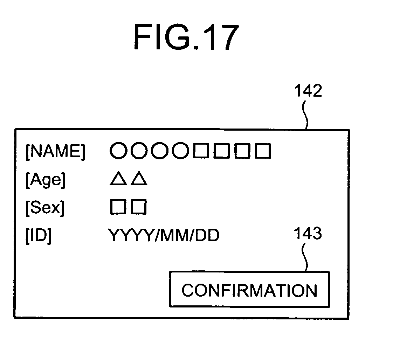

FIG. 15 is a schematic diagram of a configuration of a filing system according to a fourth embodiment; FIG. 16 is a flowchart for explaining a search operation of patient information in the filing system according to the fourth embodiment; and FIG. 17 is a diagram illustrating one example of the patient information to be displayed on the display unit 140 of the display apparatus 104. In FIG. 15, the fourth embodiment differs from the third embodiment in that a confirmation controller 153 is provided in the control unit 150 of the display apparatus 104 as a confirmation controller for controlling the confirmation of the patient information. As shown in FIG. 17, the confirmation controller 153 displays, in the display area 142 of the display unit 140, name [NAME], age [Age], sex [Sex], and examination ID [ID] as the patient information which shows characteristics of the subject, and controls to display a confirmation button 143 which allows the user to confirm the patient information.

When the confirmation button 143 is right-clicked on by operating the mouse of the input unit 120 to move the cursor on the screen, confirmation information indicating that the patient information is confirmed is input to the confirmation controller 153. When the confirmation information is input, the confirmation controller 153 determines that the user has confirmed the patient information.

Next, an operation for searching patient information in the filing system will be explained with reference to the flowchart in FIG. 16. In FIG. 16, when the user inputs one piece of patient information "Hanako Yamada" by operating the keyboard of the input unit 120 (step S501), the search unit 151, at a time when input of "Yamada" is completed (step S502), the selector 152 selects the database 130 in the display apparatus as a search target in the same way as in the third embodiment (step S503).

Next, in response to the selection of the database 130 by the selector 152, the search unit 151 searches the database 130 for patient information including the name of "Yamada", which is searched right before the current search, based on the history information (step S504), and displays the searched patient information and the confirmation button 143 (see FIG. 17) in the display area of the display unit 140 (step S505).

When the mouse of the input unit 120 is operated to move the cursor on the screen, and a confirmation operation of right-clicking on the confirmation button 143 is performed (step S506), only the patient information is displayed on the screen (step 5507).

Here, when the search unit 151 cannot find relevant patient information, the selector 152 selects the database 131 as a search target at step S503. This selection-information is transmitted to the server 106 via the interface 160. Then, in response to the selection of the database 131 by the selector 152, the search unit 171 of the server 106 searches the database 131 for patient information including the name of "Yamada", which is searched right before the current search, based on the history information (step S504), and transmits the search result of patient information to the display apparatus 104 via the interface 161.

When the search unit 151 of the display apparatus 104 retrieves the patient information, the searched patient information and the confirmation button 143 are displayed in the display area of the display unit 140 (step S505). When the mouse of the input unit 120 is operated to move the cursor on the screen, and a confirmation operation of right-clicking on the confirmation button 143 is performed (step S506), only the patient information is displayed on the screen (step S507), and the patient information is stored in the database 130 (step S508). In the fourth embodiment, when there is no relevant patient name "Hanako Yamada", all pieces of patient information such as the patient name, age, sex, examination ID, and the like are input and stored in the database 130 (step S508).

Thus in the fourth embodiment, since the searched patient information is confirmed, it is possible to prevent a mistake in selecting and handling patient information and to improve the reliability of the searched patient information with the same advantages as in the third embodiment.

In the second to fourth embodiments, the display apparatus 104 having the function especially as a filing device is explained. However, the display apparatus 104 may be combined with the function of the image display in the image display apparatus 4 according to the first embodiment. In the first embodiment, the image display apparatus 4 having the function especially of displaying images is explained. However, the display apparatus 4 may be combined with the function as a filing device in the display apparatus 104 according to the second to fourth embodiments.

### INDUSTRIAL APPLICABILITY

The image display apparatus according to the present invention is useful as an image display apparatus which displays a series of images captured at multiple time points, more specifically as an image display apparatus which displays a series of intra-subject images captured by using a capsule endoscope inserted in a subject.

## Claims

1. An image display apparatus for displaying a series of images captured at multiple time points and a time scale indicating an imaging period of the series of images, comprising:
an appending unit that appends marker information indicating an image of interest to a predetermined image among the series of images;
a display controller that controls to display one of display areas on the time scale corresponding to before and after an imaging time point of the image of interest to which the marker information is appended, and to display other display areas on the time scale so as to be discriminable from the one of the display areas.

2. The image display apparatus according to claim 1, wherein the display controller controls to display a display area on the time scale corresponding to between imaging time points of images of interest to each of which the marker information is appended, and to display other display areas on the time scale so as to be discriminable from the display area, when a plurality of images of interest to each of which the marker information is appended are present.

3. The image display apparatus according to claim 1 or 2, wherein the display controller controls to display the display areas on the time scale, each of the display areas, corresponding to before and after the imaging time point of the image of interest, having different at least one of hue, color saturation, luminance, pattern, shape, and size thereof.

4. The image display apparatus according to any one of claims 1 to 3, wherein the display controller displays respective images of interest appended with the marker information as thumbnails, and controls to display relative times corresponding to imaging time points of thumbnails in the neighborhood of the displayed thumbnails.

5. The image display apparatus according to claim 4, wherein the display controller controls to display the relative time based on an imaging time point of a reference thumbnail as a reference, the reference thumbnail being selected among the thumbnails.

6. The image display apparatus according to claim 5, further comprising:
a selection-information acquiring unit that acquires selection-information for selecting the reference thumbnail among the thumbnails, wherein
the display controller controls to update the reference for each acquisition of the selection-information by the selection-information acquiring unit, and to display the relative time.

7. The image display apparatus according to any one of claims 1 to 6, wherein the series of images are intra-subject images captured by using a capsule endoscope inserted into a subject.
